# EUROPEAN PATENT APPLICATION

(11) **EP 4 817 583 A1**
(43) Date of publication of application: **07.10.2026**
(21) Application number: 25738889.2
(22) Date of filing: 06.01.2025
(51) Int. Cl.: A61H 5/00, G06F 3/01, G02B 27/01, A61F 9/02

(54) **VISUAL PERCEPTION LEARNING-BASED APPARATUS AND METHOD FOR PROVIDING STEREOVISION TRAINING**

(30) Priority: 08.01.2024 KR 20240002923
(71) Applicant: The Asan Foundation, Seoul 05505 (KR); NUNAPS INC., Seoul 06101 (KR); UNIVERSITY OF ULSAN FOUNDATION FOR INDUSTRY COOPERATION, Nam-gu Ulsan 44610 (KR)
(72) Inventor: KANG, Dong Wha, Seoul 05505 (KR); LEE, Byung Joo, Seoul 05505 (KR); RYU, Young Hoe, Seoul 05505 (KR); PARK, In Seok, Seoul 05505 (KR)
(74) Representative: dompatent
(86) International application number: PCT/KR2025/000235
(87) International publication number: WO 2025/150815

(57) **Abstract**

The present disclosure relates to an apparatus and method for providing visual perception learning-based stereoscopic vision training, wherein the apparatus may include a display module, a memory configured to store at least one process for strabismus improvement based on stereoscopic vision training, and a processor configured to generate a first stimulus plate for a normal eye among both eyes of a trainee and a second stimulus plate for a strabismic eye among the both eyes, and configured to provide the stereoscopic vision training through difficulty adjustment of a first stimulus among a plurality of stimuli included in each of the first stimulus plate and the second stimulus plate, wherein the difficulty adjustment may be performed through at least one of stereoscopic effect setting of a stimulus, shape setting of a stimulus, interval setting of a stimulus, border setting of a stimulus, and background color setting of a stimulus.

## Description

### [TECHNICAL FIELD]

The present disclosure relates to an apparatus and method for providing visual perception learning-based stereoscopic vision training.

### [BACKGROUND ART]

Recently, due to development of media, incidence rates of myopia, astigmatism, strabismus, and the like are increasing. Among them, strabismus refers to a state in which two eyes are not aligned straight, and if not treated early, vision may not be permanently recovered, and thus should be detected and corrected early during childhood.

If strabismus occurs at around 2 to 5 years of age, two eyes of a corresponding child patient see different objects from each other, and accordingly, two completely different images are transmitted to the brain, thereby causing visual confusion in which completely different objects appear to overlap each other. In addition, as a diplopia phenomenon in which one image appears to exist at different locations simultaneously is perceived, the child patient ignores an image entering from one side in order to resolve visual confusion and diplopia, and consequently, the child patient abandons binocular vision capability enabling stereoscopic perception of an object, and furthermore abandons use of an eye having strabismus, thereby even causing amblyopia.

Accordingly, there is a need to develop a technology for providing and controlling stereoscopic vision training in order to improve visual perception capability including stereoscopic sense of a user (trainee).

### [DETAILED DESCRIPTION OF THE INVENTION]

### [TECHINICAL PROBLEM]

An embodiment disclosed in the present disclosure is to provide an apparatus and method for providing visual perception learning-based stereoscopic vision training.

Problems to be solved by the present disclosure are not limited to the problems mentioned above, and other unmentioned problems will be clearly understood by a person who is ordinary skilled in the art from the following description.

### [TECHNICAL SOLUTION]

In an aspect, an apparatus for providing visual perception learning-based stereoscopic vision training according to the present disclosure may include a display module, a memory configured to store at least one process for strabismus improvement based on stereoscopic vision training, and a processor configured to generate a first stimulus plate for a normal eye among both eyes of a trainee and a second stimulus plate for a strabismic eye among the both eyes, and configured to provide the stereoscopic vision training through difficulty adjustment of a first stimulus among a plurality of stimuli included in each of the first stimulus plate and the second stimulus plate, wherein the difficulty adjustment may be performed through at least one of stereoscopic effect setting of a stimulus, shape setting of a stimulus, interval setting of a stimulus, border setting of a stimulus, and background color setting of a stimulus.

Also, the processor may identify a strabismus angle of the strabismic eye through evaluation of a strabismus angle of the trainee, and may set positions of the first stimulus plate and the second stimulus plate within an image for the stereoscopic vision training based on the strabismus angle.

Also, the positions of the first stimulus plate and the second stimulus plate within the image may be set such that a center of the first stimulus plate and a center of the second stimulus plate differ from each other by the strabismus angle on a horizontal line.

Also, the stereoscopic effect setting of the stimulus may be a method in which a difficulty level is higher as a distance by which the first stimulus included in the second stimulus plate moves with respect to a center of the second stimulus plate is smaller, and the shape setting of the stimulus may be a method in which a difficulty level is higher as a shape has less symmetry.

Also, the interval setting of the stimulus may be a method in which a difficulty level is higher as a distance between the first stimulus and a second stimulus among the plurality of stimuli is greater, and the background color setting of the stimulus may be a method in which a difficulty level is higher as a difference between the background color and colors of the first stimulus and the second stimulus is smaller.

Also, the border setting of the stimulus may be a method in which a difficulty level is higher as a distance between a border set by a first method or a second method and at least one of the first stimulus and the second stimulus is greater, wherein the first method may comprise setting borders for the first stimulus and the second stimulus, respectively, and the second method may comprise setting a border including the first stimulus and the second stimulus.

Also, the processor may set a minimum border formation condition based on an angle for the stereoscopic effect setting, and may apply the minimum border formation condition when performing the border setting of the stimulus.

Also, the processor may set priorities of the interval setting of the stimulus, the border setting of the stimulus, and the background color setting of the stimulus by using an artificial intelligence model trained with training results of other trainees.

Also, the processor may provide the stereoscopic vision training by providing a third stimulus based on auditory sensation or tactile sensation in a form associated with the first stimulus.

Also, in another aspect, a method for providing visual perception learning-based stereoscopic vision training performed by a processor of an apparatus of the present disclosure may include generating, by the processor, a first stimulus plate for a normal eye among both eyes of a trainee and a second stimulus plate for a strabismic eye among the both eyes, and providing, by the processor, stereoscopic vision training through difficulty adjustment of a first stimulus among a plurality of stimuli included in each of the first stimulus plate and the second stimulus plate, wherein the difficulty adjustment may be performed through at least one method of stereoscopic effect setting of a stimulus, shape setting of a stimulus, interval setting of a stimulus, border setting of a stimulus, and background color setting of a stimulus.

In addition, a computer program stored in a computer-readable recording medium for executing a method for implementing the present disclosure may be further provided.

In addition, a computer-readable recording medium recording a computer program for executing a method for implementing the present disclosure may be further provided.

### [ADVANTAGEOUS EFFECTS OF THE INVENTION]

According to the above-described means for solving the problems of the present disclosure, it is possible to train stereoscopic vision of a trainee by setting stimulus plates provided to both eyes based on a strabismus angle identified through strabismus angle evaluation and providing a stereoscopic effect to a stimulus included in the stimulus plates.

Also, it is possible to improve an effect of stereoscopic vision training by adjusting a difficulty level of a stereoscopic stimulus in which a stereoscopic effect is formed in various ways.

Effects of the present disclosure are not limited to the above-mentioned effects, and other unmentioned effects will be clearly understood by a person having ordinary skill in the art from the description.

### [DESCRIPTION OF THE DRAWINGS]

FIG. 1 is a diagram illustrating a block diagram of an apparatus for providing visual perception learning-based stereoscopic vision training according to an embodiment of the present disclosure.
FIG. 2 is a flowchart of a method for providing visual perception learning-based stereoscopic vision training according to an embodiment of the present disclosure.
FIG. 3 and FIG. 4 are diagrams for describing a difference between a normal eye and a strabismic eye according to an embodiment of the present disclosure.
FIG. 5A and FIG. 5B are diagrams for describing strabismus angle measurement and stimulus plate setting according to an embodiment of the present disclosure.
FIG. 6A and FIG. 6B are diagrams for describing application of a stereoscopic effect to a stereoscopic stimulus for stereoscopic vision training according to an embodiment of the present disclosure.
FIG. 7 is a diagram for describing a stereoscopic effect level according to an embodiment of the present disclosure.
FIG. 8A and FIG. 8B are diagrams for describing border setting according to an embodiment of the present disclosure.
FIG. 9 is a diagram for describing a minimum border formation condition according to an embodiment of the present disclosure.

### [BEST MODE]

Throughout the present disclosure, identical reference numerals refer to identical components. The present disclosure does not describe all elements of the embodiments, and general contents in the technical field to which the present disclosure pertains or overlapping contents among the embodiments are omitted. The terms 'part', 'module', 'member', and 'block' used in the specification may be implemented by software or hardware, and according to embodiments, a plurality of 'parts', 'modules', 'members', and 'blocks' may be implemented as one component, or one 'part', 'module', 'member', and 'block' may include a plurality of components. Throughout the specification, when a certain part is "connected" to another part, this includes not only a case in which the certain part is directly connected to the another part but also a case in which the certain part is indirectly connected to the another part, and the indirect connection includes connection through a wireless communication network.

Also, when a certain part "includes" a certain component, this means that another component may be further included rather than excluding another component unless specifically stated otherwise.

Throughout the specification, when a certain member is positioned "on" another member, this includes not only a case in which the certain member is in contact with the another member but also a case in which another member exists between the two members.

Terms such as first and second are used for distinguishing one component from another component, and the component is not limited by the above-described terms.

A singular expression includes a plural expression unless clearly indicated otherwise by context.

In each step, an identification symbol is used for convenience of description, and the identification symbol does not describe an order of each step, and each step may be performed differently from a stated order unless a specific order is clearly stated by context.

Hereinafter, operation principles and embodiments of the present disclosure will be described with reference to the accompanying drawings.

In the present specification, "apparatus" includes all kinds of apparatuses capable of performing arithmetic processing and providing a result to a user. For example, an apparatus according to the present disclosure may include all of a computer, a server apparatus, and a portable terminal, or may be any one form thereof.

Herein, the computer may include, for example, a notebook equipped with a WEB browser, a desktop, a laptop, a tablet PC, a slate PC, and the like.

The server apparatus is a server processing information by performing communication with an external apparatus, and may include an application server, a computing server, a database server, a file server, a game server, a mail server, a proxy server, a web server, and the like.

The portable terminal may include, for example, a wireless communication apparatus having portability and mobility, and may include all types of handheld-based wireless communication apparatuses such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), a WiBro (Wireless Broadband Internet) terminal, and a smart phone (Smart Phone), and wearable apparatuses such as a watch, a ring, a bracelet, an anklet, a necklace, glasses, contact lenses, or a head-mounted-device (HMD).

A function related to artificial intelligence according to the present disclosure operates through a processor and a memory. The processor may be configured as one processor or a plurality of processors. In this case, the one processor or the plurality of processors may be a general-purpose processor such as a CPU, an AP, and a DSP (Digital Signal Processor), a graphics-dedicated processor such as a GPU and a VPU (Vision Processing Unit), or an artificial intelligence-dedicated processor such as an NPU. The one processor or the plurality of processors control processing of input data according to predefined operation rules or an artificial intelligence model stored in the memory. Alternatively, when the one processor or the plurality of processors are artificial intelligence-dedicated processors, the artificial intelligence-dedicated processor may be designed with a hardware structure specialized for processing of a specific artificial intelligence model.

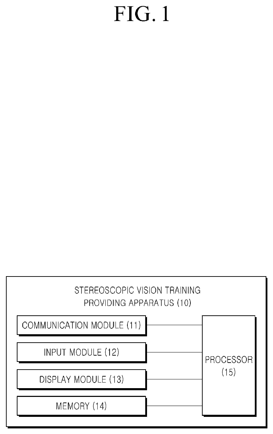

FIG. 1 is a diagram illustrating a block diagram of an apparatus for providing visual perception learning-based stereoscopic vision training according to an embodiment of the present disclosure.

Referring to FIG. 1, an apparatus for providing visual perception learning-based stereoscopic vision training (hereinafter, stereoscopic vision training apparatus) 10 may include a communication module 11, an input module 12, a display module 13, a memory 14, and a processor 15. Components illustrated in FIG. 1 are not essential in implementing the stereoscopic vision training providing apparatus 10 according to the present disclosure, and thus the stereoscopic vision training providing apparatus 10 described in the present specification may have more or fewer components than the above-listed components.

The stereoscopic vision training providing apparatus 10 according to an embodiment of the present disclosure may include various apparatuses capable of performing arithmetic processing. For example, the stereoscopic vision training providing apparatus 10 may include a desktop PC, a mobile phone, a smart phone, a laptop computer, a PDA (personal digital assistants), a PMP (portable multimedia player), a slate PC, a tablet PC, an ultrabook, a wearable device, and the like.

Meanwhile, the stereoscopic vision training providing apparatus 10 may include a display apparatus such as a mobile phone capable of being mounted on a head-mounted device such as an HMD (Head Mounted Display), smart glasses, and smart goggles mounted on a head of a trainee to display an image.

The communication module 11 performs wired or wireless communication with at least one external apparatus (server, etc.). Particularly, in performing wireless communication, the communication module 11 transmits and receives wireless signals in a communication network according to wireless Internet technologies.

Examples of the wireless Internet technologies may include WLAN (Wireless LAN), Wi-Fi (Wireless-Fidelity), Wi-Fi (Wireless Fidelity) Direct, DLNA (Digital Living Network Alliance), WiBro (Wireless Broadband), WiMAX (World Interoperability for Microwave Access), HSDPA (High Speed Downlink Packet Access), HSUPA (High Speed Uplink Packet Access), LTE (Long Term Evolution), LTE-A (Long Term Evolution-Advanced), and the like, and the stereoscopic vision training providing apparatus 100 transmits and receives data according to at least one wireless Internet technology within a range including even Internet technologies not listed above.

As being for short range communication, short range communication may be supported by using at least one of Bluetooth (Bluetooth^{™}), RFID (Radio Frequency Identification), infrared communication (Infrared Data Association; IrDA), UWB (Ultra Wideband), ZigBee, NFC (Near Field Communication), Wi-Fi (Wireless-Fidelity), Wi-Fi Direct, and Wireless USB (Wireless Universal Serial Bus) technologies. In this case, a short range wireless communication network may be a Wireless Personal Area Network.

The input module 12 may acquire a signal corresponding to an input of a trainee. For example, the input module 12 may acquire an input of the trainee for strabismus angle measurement or performance of stereoscopic vision training, a response to a strabismus angle evaluation operation and a stereoscopic vision training operation provided through the display module 13, and the like.

In this case, the input module 12 may include a keyboard, a keypad, a button, a jog shuttle, a wheel, and the like. Also, the input of the trainee in the input module 12 may be, for example, pressing of a button, touch, drag, and the like.

The input module 12 may be configured as a separate module connected to the stereoscopic vision training providing apparatus 10 wirelessly or wiredly. For example, the stereoscopic vision training providing apparatus 10 may provide, to a trainee, an image for performing strabismus angle evaluation or stereoscopic vision training through the display module 13 mounted and attached to a head of the trainee, and may receive a response from the trainee through the input module 12 configured as a separate module provided to a hand of the trainee.

The display module 13 outputs an image or a picture. For example, the display module 13 may include an LCD, an OLED, an AMOLED display, and the like. In an embodiment, when the display module 13 is implemented as a touch screen, the display module 13 may also perform a role of the input module 12. In this case, according to selection, a separate input module 12 may not be provided, or the input module 12 performing limited functions such as volume adjustment, a power button, and a home button may be provided. Also, the display module 13 may be provided in a form of an image output port transmitting image information to an external display apparatus.

The display module 13 may include a plurality of first displays and second displays respectively corresponding to both eyeballs of a trainee. Here, the first display may output a first image, and the second display may output a second image.

Meanwhile, the present disclosure is not limited thereto, and the display module 13 may be implemented as one display. In this case, the display module 13 may output the first image to a left area of the display and may output the second image to a right area of the display.

The memory 14 stores at least one piece of data (information) and at least one process necessary for strabismus improvement based on stereoscopic vision training, as well as various kinds of data (information). For example, the memory 14 may store, as data, a program for providing and performing training, at least one piece of trainee information (personal information, visual information, response information, training result, etc.), various reference values serving as references for strabismus angle evaluation, a measurement image, a training image, and the like. In addition, the memory 14 may store various commands, algorithms, and the like for executing a strabismus improvement method based on stereoscopic vision training.

Also, the memory 14 may include a storage medium of at least one type among a flash memory type, a hard disk type, a multimedia card micro type, a card-type memory (e.g., SD memory or XD memory), RAM (Random Access Memory), SRAM (Static Random Access Memory), ROM (Read-Only Memory), EEPROM (Electrically Erasable Programmable Read-Only Memory), PROM (Programmable Read-Only Memory), a magnetic memory, a magnetic disk, and an optical disk. In addition, the memory 14 may store information temporarily, permanently, or semi-permanently, and may be provided in an embedded type or a detachable type.

The processor 15 is for controlling components within the stereoscopic vision training providing apparatus 10 or processing and calculating various kinds of information, and controls such that a strabismus improvement operation is performed based on at least one process stored in the memory 14.

Before performing the strabismus improvement operation, the processor 15 may perform a stimulus plate setting operation through evaluation of a strabismus angle of a trainee. Specifically, the processor 15 may output a first reference image to an area corresponding to a left eye and may output a second reference image to an area corresponding to a right eye through the display module 13. In this case, the first reference image and the second reference image may be output such that a specific shape (e.g., a quadrangle, a heart, etc.) is visible when viewed together based on a normal eye. Meanwhile, when the first reference image and the second reference image are viewed together based on a strabismic eye, left-right spacing may exist, and thus the specific shape described above may not be visible. In this case, the trainee may complete the specific shape by moving the first reference image or the second reference image in a left-right direction through the input module 12. Through this, the processor 15 may calculate which one of the left eye and the right eye of the trainee is a strabismic eye and may calculate a degree of the strabismus angle. Also, the processor 15 may generate a stimulus plate based on the calculated strabismus angle. For example, the processor 15 may set a position of the stimulus plate such that an image for stereoscopic vision training is output through the display module 13 to correspond to a viewing angle of each of the normal eye and/or the strabismic eye of the trainee by using the calculated strabismus angle.

Also, the processor 15 may perform a stereoscopic vision training operation (strabismus improvement operation) based on the set position of the stimulus plate. The stereoscopic vision training operation is training for allowing a trainee to select a stereoscopic stimulus among a planar stimulus and the stereoscopic stimulus. In this case, the processor 15 may allow training for strabismus improvement of the trainee to be efficiently performed by changing a difficulty level of the stereoscopic stimulus in various ways.

The processor 15 may be implemented by software, hardware, and a combination thereof. For example, in terms of hardware, the processor 15 may be implemented as an FPGA (field programmable gate array), an ASIC (Application Specific Integrated Circuit), a semiconductor chip, and various other forms of electronic circuits. Also, for example, in terms of software, the processor 15 may be implemented as a logic program, various computer languages, and the like performed according to the above-described hardware.

In the following description, unless otherwise stated, an operation of the stereoscopic vision training providing apparatus 10 may be understood as being performed under control of the processor 15.

As described above, the stereoscopic vision training providing apparatus 10 according to the present disclosure may determine a position of a stimulus based on an angle from a lens center rather than setting the position of the stimulus based on an orthogonal coordinate system by preliminarily performing a stimulus plate position setting operation through strabismus angle evaluation. Accordingly, by accurately reflecting a degree of a strabismus angle according to a condition of a trainee at a time of training and accurately providing a stimulus at a strabismus angle position, an effect of stereoscopic vision training can be improved.

Also, the stereoscopic vision training providing apparatus 10 according to the present disclosure can improve an effect of stereoscopic vision training of a trainee by providing not only static stereoscopic vision training but also dynamic stereoscopic vision training together.

Meanwhile, according to an embodiment, the stereoscopic vision training providing apparatus 10 may be implemented to perform a strabismus angle evaluation operation and a stereoscopic vision training operation through an external apparatus. For example, when the stereoscopic vision training providing apparatus 100 is implemented as a server, the stereoscopic vision training providing apparatus 100 may communicate with an external apparatus through the communication module 110 to perform a stimulus plate generation operation and a stereoscopic vision training operation (strabismus improvement operation). In this case, an operation using the input module 12 and the display module 13 of the stereoscopic vision training providing apparatus 10 described in the present specification may be replaced with an operation using a display module and an input module of the external apparatus through the communication module 11.

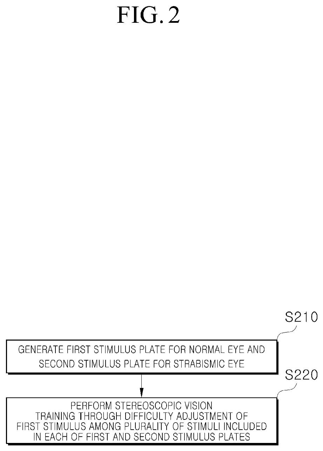

FIG. 2 is a flowchart of a method for providing visual perception learning-based stereoscopic vision training according to an embodiment of the present disclosure.

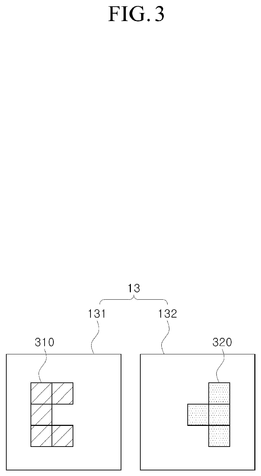

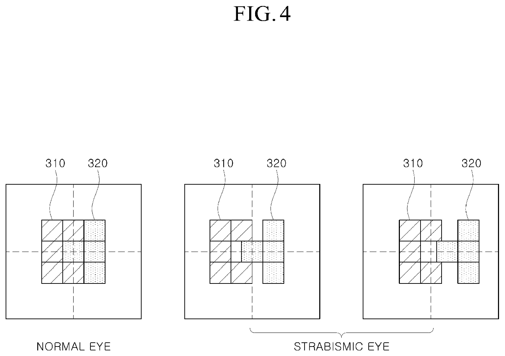

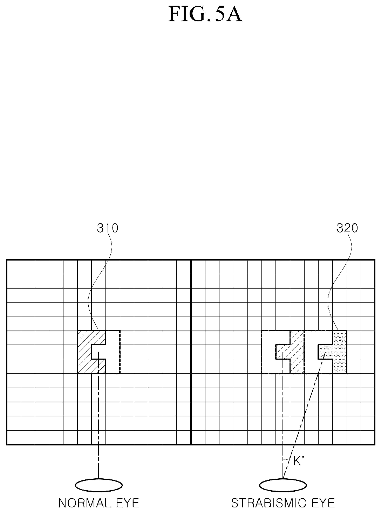

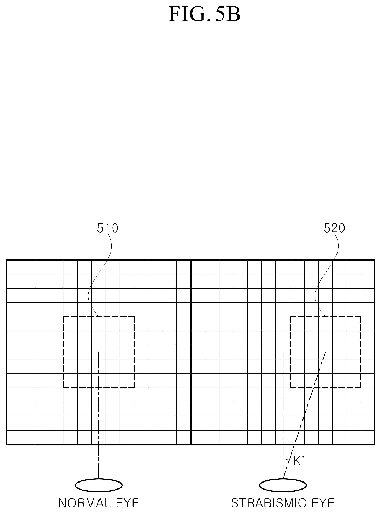

FIG. 3 and FIG. 4 are diagrams for describing a difference between a normal eye and a strabismic eye according to an embodiment of the present disclosure. FIG. 5A and FIG. 5B are diagrams for describing strabismus angle measurement and stimulus plate setting according to an embodiment of the present disclosure.

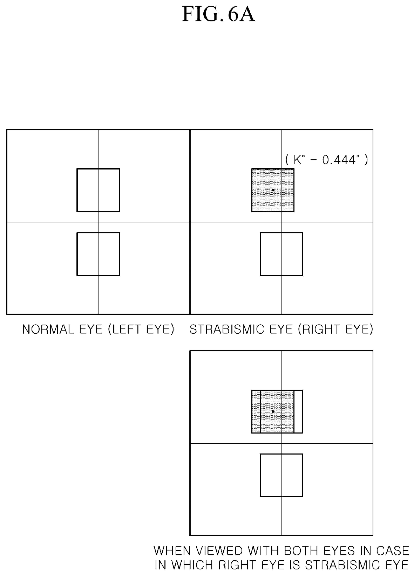

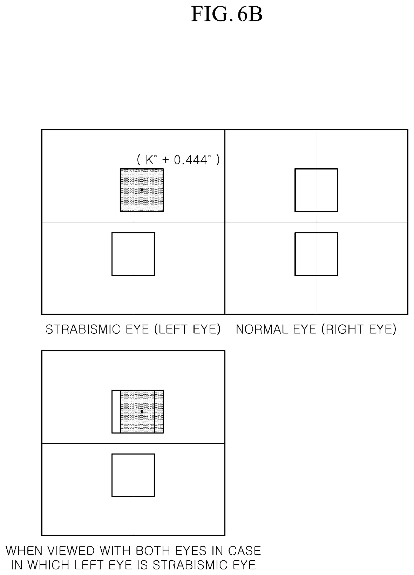

FIG. 6A and FIG. 6B are diagrams for describing application of a stereoscopic effect to a stereoscopic stimulus for stereoscopic vision training according to an embodiment of the present disclosure.

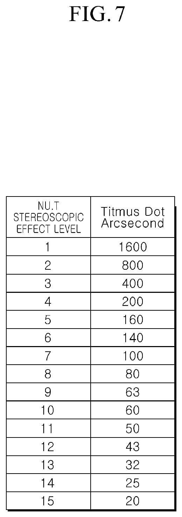

FIG. 7 is a diagram for describing a stereoscopic effect level according to an embodiment of the present disclosure.

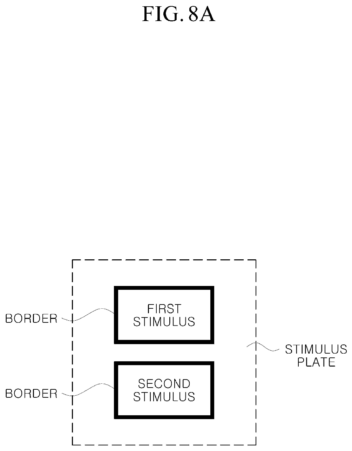

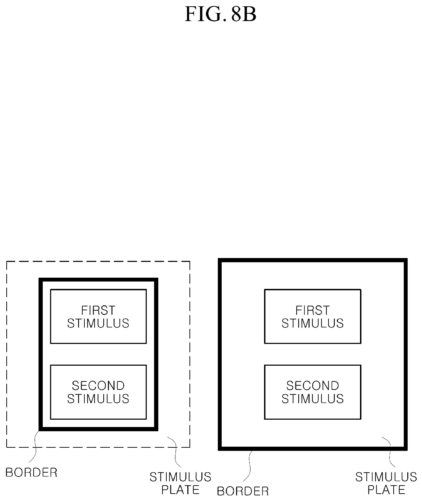

FIG. 8A and FIG. 8B are diagrams for describing border setting according to an embodiment of the present disclosure.

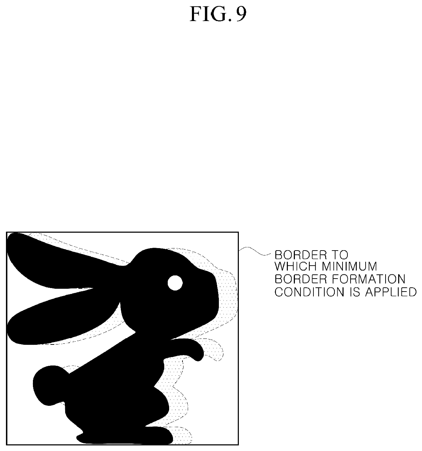

FIG. 9 is a diagram for describing a minimum border formation condition according to an embodiment of the present disclosure.

Before describing a strabismus improvement method based on stereoscopic vision training according to an embodiment of the present disclosure with reference to FIG. 2, a difference between a normal eye and a strabismic eye according to an embodiment of the present disclosure will be described with reference to FIG. 3 and FIG. 4.

Referring to FIG. 3, the display module 13 may include a first display 131 and a second display 132. In the present disclosure, the first display 131 and the second display 132 may be implemented as a plurality of physically separated displays or may be implemented as a concept of areas divided into a left area and a right area within one display.

In an embodiment, the first display 131 may output a first reference image 310 corresponding to a left eye, and the second display 132 may output a second reference image 320 corresponding to a right eye. The first reference image 310 and the second reference image 320 may be different images from each other. For example, referring to FIG. 3, the first reference image 310 is an image having a '⊏' shape, and the second reference image 320 is an image having a '┤' shape.

Also, the first reference image 310 and the second reference image 320 may be output such that the first reference image 310 and the second reference image 320 appear to form a specific shape together when viewed by a trainee having both eyes as normal eyes. For example, referring to FIG. 4, when viewed by a trainee having both eyes as normal eyes, the first reference image 310 and the second reference image 320 may be output to appear as a quadrangle formed by combining '⊏' and '┤'. Such a specific shape is hereinafter referred to as a reference shape.

Meanwhile, when a trainee having one strabismic eye among both eyes views the first reference image 310 and the second reference image 320, the images will appear differently from the above. For example, referring to FIG. 4, the trainee having one strabismic eye among both eyes will perceive the first reference image 310 or the second reference image 320 as being more spaced apart to a left side or a right side, and will not perceive the images as a quadrangle formed by combining '⊏' and '┤'.

The trainee may move eyeballs or move a head such that the first reference image 310 and the second reference image 320 have the reference shape. The processor 15 may determine a strabismic eye among both eyes of the trainee based on a distance by which the eyeballs or the head move, and may determine a strabismus angle of the strabismic eye.

Meanwhile, shapes of the first reference image 310 and the second reference image 320 are not limited to the examples illustrated in FIG. 3 and FIG. 4, and may be implemented as various images. Also, although FIG. 4 and FIG. 5 illustrate and describe the first reference image 310 and the second reference image 320 as different images from each other, the present disclosure is not limited thereto, and the first reference image 310 and the second reference image 320 may be implemented as identical images.

Hereinafter, with reference to FIG. 2 and FIG. 5 to FIG. 9, a stimulus plate generation operation through strabismus angle evaluation and a stereoscopic vision training operation will be described.

Referring to FIG. 2, the processor 15 may generate a first stimulus plate for a left eye among both eyes of a trainee and a second stimulus plate for a right eye among the both eyes (S210).

The processor 15 may identify a strabismic eye having strabismus among both eyes through evaluation of a strabismus angle of the trainee, and may also identify a strabismus angle of the strabismic eye. Hereinafter, unless otherwise stated, description will be given using, as an example, a case in which the right eye is a strabismic eye, and the same description may be applied even in a case in which the left eye is a strabismic eye.

Here, as described above, the strabismus angle evaluation may be performed based on a distance by which eyeballs or a head of the trainee move when the first reference image 310 and the second reference image 320 are provided to the trainee through the display module 13.

The processor 15 may set positions of the first stimulus plate and the second stimulus plate within an image for the stereoscopic vision training (hereinafter, training image) based on the calculated strabismus angle.

Specifically, positions of the first stimulus plate within a first training image and the second stimulus plate within a second training image may be set such that a center of the first stimulus plate and a center of the second stimulus plate differ from each other by the strabismus angle on a horizontal line. Here, the first training image may be an image for a left eye, and the second training image may be an image for a right eye.

That is, the processor 15 may set a position of the first stimulus plate within the first training image and a position of the second stimulus plate within the second training image such that an angle between the center of the first stimulus plate and the center of the second stimulus plate is identical to the strabismus angle.

Referring to FIG. 5A, when '┤' within the second reference image moves by k° with respect to 0° and becomes the reference shape formed by combining '⊏' and '┤', the strabismus angle may be k°.

Referring to FIG. 5B, when a strabismic eye is a right eye, the processor 15 may set a first stimulus plate 510 of an eye (left eye) that is not the strabismic eye based on 0°, and may set a second stimulus plate 520 of an eye (right eye) that is the strabismic eye based on k°. That is, an angle between a center of the first stimulus plate 510 and a center of the second stimulus plate 520 may be k°.

Accordingly, during stereoscopic vision training, an image for a normal eye and an image for a strabismic eye provided to a trainee may be set such that stimulus plates are positioned based on 0° and k°, respectively.

The processor 15 may provide (perform) stereoscopic vision training through difficulty adjustment of a first stimulus among a plurality of stimuli included in each of the first stimulus plate and the second stimulus plate (220).

Here, the first stimulus may be a stereoscopic stimulus, and the second stimulus may be a planar stimulus.

That is, when two objects (a stereoscopic stimulus and a planar stimulus) are respectively included in a first training image for a left eye and a second training image for a right eye, and the right eye is a strabismic eye, when the processor 15 maintains a position of the stereoscopic stimulus included in the first training image for the left eye as it is and adjusts a position of the stereoscopic stimulus included in the second training image for the right eye, a stereoscopic effect is formed in the stereoscopic stimulus. The trainee simultaneously views the stereoscopic stimulus in which the stereoscopic effect is formed and the planar stimulus without the stereoscopic effect, thereby recognizing the stereoscopic effect of the stereoscopic stimulus and providing (performing) stereoscopic vision training.

In the present disclosure, difficulty adjustment of the first stimulus (stereoscopic stimulus) may be performed through at least one of stereoscopic effect setting of a stimulus, shape setting of a stimulus, interval setting of a stimulus, border setting of a stimulus, and background color setting of a stimulus.

The stereoscopic effect setting of the stimulus is a method of adjusting a difficulty level by setting a stereoscopic effect based on a distance by which the first stimulus included in the second stimulus plate within the second training image moves with respect to a center of the second stimulus plate. Specifically, the difficulty level may be higher as the distance by which the first stimulus included in the second stimulus plate moves with respect to the center of the second stimulus plate is smaller. In this case, a shape of the stimulus is not limited to one form, and various forms may be applied. This will be described later.

Referring to FIG. 6A, in a case in which a left eye is a normal eye and a right eye is a strabismic eye, a stereoscopic stimulus is positioned at an upper side of a first stimulus plate for the left eye, a planar stimulus is positioned at a lower side thereof, a stereoscopic stimulus is positioned at an upper side of a second stimulus plate for the right eye, and a planar stimulus is positioned at a lower side thereof. In this case, as described above, a center of the first stimulus plate is based on 0°, and a center of the second stimulus plate is based on k°. The processor 15 may move the stereoscopic stimulus included in the second stimulus plate leftward by 0.444° from k° for a stereoscopic effect of the stereoscopic stimulus. Accordingly, when viewed with both eyes, the trainee can identify the stereoscopic stimulus to which a stereoscopic effect of -0.444° is applied in comparison with the planar stimulus.

FIG. 6B illustrates a case in which a left eye is a strabismic eye and a right eye is a normal eye. Referring to FIG. 6B, a stereoscopic stimulus is positioned at an upper side of a first stimulus plate for the left eye, a planar stimulus is positioned at a lower side thereof, a stereoscopic stimulus is positioned at an upper side of a second stimulus plate for the right eye, and a planar stimulus is positioned at a lower side thereof. In this case, a center of the first stimulus plate is based on k°, and a center of the second stimulus plate is based on 0°. The processor 15 may move the stereoscopic stimulus included in the first stimulus plate rightward by 0.444° from k° for a stereoscopic effect of the stereoscopic stimulus. Accordingly, when viewed with both eyes, the trainee can identify the stereoscopic stimulus to which a stereoscopic effect of +0.444° is applied in comparison with the planar stimulus.

Referring to FIG. 7, as a stereoscopic effect level increases, an arcsec unit value indicating the stereoscopic effect decreases. Here, 1600 arcsec is 0.444°. That is, when the arcsec unit value is lowered according to the stereoscopic effect level, an angle by which the stereoscopic stimulus included in the stimulus plate for the strabismic eye moves becomes correspondingly smaller, and thus the difficulty level increases.

A stereoscopic effect setting method of a stimulus may be a basic factor largely determining a difficulty level of training. That is, after basically setting a difficulty level of a stimulus through stereoscopic effect setting, a detailed difficulty level may be adjusted by applying remaining methods.

The shape setting of the stimulus is a method of adjusting a difficulty level through a shape of the stimulus. Here, the shape of the stimulus may be variously implemented as a heart shape, a star shape, a circle shape, a tree shape, a rabbit shape, an elephant shape, a fish shape, a house shape, a butterfly shape, a car shape, and the like.

An identical shape may be applied to the stereoscopic stimulus and the planar stimulus for training. For example, both the stereoscopic stimulus and the planar stimulus may have a rabbit shape. However, the present disclosure is not limited thereto, and a shape of the stereoscopic stimulus and a shape of the planar stimulus may be differently set.

In an embodiment, in the shape setting method of the stimulus, a difficulty level may be higher as a shape has less symmetry. Specifically, a training image having top-bottom-left-right symmetry (e.g., a circle shape) is relatively easier to perceive a stereoscopic effect than an image having only top-bottom symmetry or only left-right symmetry (e.g., a star shape, a heart shape, a tree shape, and a butterfly shape) or a training image having no symmetry (a rabbit shape, an elephant shape, a fish shape, a house shape, and a car shape). In this respect, the processor 15 may set a difficulty level to gradually increase in an order of 'a training image having top-bottom-left-right symmetry - an image having top-bottom symmetry or an image having left-right symmetry - an image having no symmetry'.

According to an embodiment, the shape setting method of the stimulus may be a basic factor largely determining a difficulty level of training together with the stereoscopic effect setting method of the stimulus. That is, after basically setting a difficulty level of the stimulus through stereoscopic effect setting and shape setting, a detailed difficulty level may be adjusted by applying remaining methods.

The interval setting of the stimulus is a method of adjusting a difficulty level through adjustment of a distance between the first stimulus and the second stimulus. Specifically, the difficulty level may be higher as the distance between the first stimulus and the second stimulus is greater. This is because, as the stereoscopic stimulus and the planar stimulus are closer to each other, a trainee can more easily perceive a difference in stereoscopic effect, and thus when increasing a difficulty level of training, a distance between stimulus objects within a stimulus plate is set to be greater.

The background color setting of the stimulus is a method of adjusting a difficulty level through adjustment of a background color of a stimulus plate. Specifically, the difficulty level may be higher as a difference between the background color of the stimulus plate and colors of the first stimulus and the second stimulus is smaller.

In an embodiment, the processor 15 may set colors of portions excluding the first stimulus and the second stimulus in the first and second stimulus plates to colors opposed to colors of the first and second stimuli (e.g., complementary colors). Accordingly, a trainee can perceive a stereoscopic effect of the stimulus more greatly.

Conversely, the processor 15 may set colors of portions excluding the first and second stimuli in the first and second stimulus plates to colors similar to colors of the first and second stimuli (e.g., colors of the same series).

In another embodiment, the processor 15 may provide background colors of the first and second stimulus plates in a form including a pattern rather than a single color. According to similarity between a pattern of the background color and colors of the stimulus, the background color including the pattern may become a hint factor or an obstacle factor in training. As the pattern of the background color and the colors of the stimulus are more similar, the trainee will feel more difficulty in training, and as a difference between the pattern of the background color and the colors of the stimulus becomes greater, the trainee will perceive a stereoscopic effect more greatly.

The border setting of the stimulus may adjust a difficulty level through border setting based on a first method or a second method.

Here, the first method may be a method of setting borders for the first stimulus and the second stimulus, respectively. Referring to FIG. 8A, the processor 15 may set individual borders for each of the first stimulus and the second stimulus included in each of the first stimulus plate and the second stimulus plate.

The second method may be a method of setting a border including the first stimulus and the second stimulus. Referring to FIG. 8B, the processor 15 may set a border to encompass the first stimulus and the second stimulus, or may set a border on the stimulus plate itself.

Specifically, in the border setting method of the stimulus, when a border is set according to the first method, the difficulty level may be higher as a distance between a border set for the first stimulus and the first stimulus and a distance between a border set for the second stimulus and the second stimulus are greater, and when a border is set according to the second method, the difficulty level may be higher as a distance between a border set to include the first and second stimuli and the first and second stimuli is greater.

Such a method creates an effect of forming a plane at a specific distance within a virtual space through a border, thereby making it easier to determine whether a stereoscopic effect exists based on a plane of the corresponding border rather than giving a feeling that an object (stimulus) is floating in the air.

The above-described first method is a method suitable for identifying how much a stimulus has a stereoscopic effect from a plane, and the above-described second method is a method suitable for comparing a stereoscopic effect between a stereoscopic stimulus and a planar stimulus. That is, the first method is a method of comparing a stereoscopic effect between stimuli with a plane (stimulus plate) within the plane, and the second method is a method of identifying how much more each stimulus has a stereoscopic effect than a plane (stimulus plate).

According to an embodiment, when following the first method in generating a border, the processor 15 may set a minimum border formation condition based on an angle for the stereoscopic effect setting, and may apply the minimum border formation condition when performing the border setting of the stimulus.

Referring to FIG. 9, when individual borders are set for respective stimuli, because the borders should not have a stereoscopic effect while objects (stimuli) within the borders should have a stereoscopic effect, a border of the stereoscopic stimulus may be output at a position of the strabismus angle (k°) as it is, and the stereoscopic stimulus may be output after being moved by a distance corresponding to an angle for forming the stereoscopic effect. In this case, when the stereoscopic stimulus invades the border, when an object is fused by both eyes, an input stimulus is abnormally perceived as protruding outside the border, and thus a situation in which trainees can easily distinguish the stereoscopic stimulus may occur. In order to prevent such a situation, the minimum border formation condition is set.

That is, because an effect in which a difficulty level of training becomes easier occurs as the border becomes closer to a stimulus object, the minimum border formation condition is set according to a stereoscopic effect level. For example, when a stereoscopic effect level is set to level 1 in stereoscopic effect setting and the stereoscopic stimulus is moved by a distance of 0.444° (1600 arcsec), the processor 15 may set, as the minimum border formation condition, a condition in which the stimulus object and the border are spaced apart by at least a distance obtained by moving 0.444° based on k°.

According to an embodiment, the processor 15 may finely adjust a difficulty level while adjusting a distance from a center of a stimulus object to a border, but the distance from the center to the border should be set to a value satisfying the minimum border formation condition.

According to an embodiment, the processor 15 may change the minimum border formation condition according to a change in a size of a stimulus object. That is, when the size of the stimulus object increases, the minimum border formation condition (minimum distance from the center to the border) may also increase.

Meanwhile, the processor 15 may set priorities of the interval setting of the stimulus, the border setting of the stimulus, and the background color setting of the stimulus by using an artificial intelligence model trained with training results of other trainees.

That is, the processor 15 may set a basic difficulty level of training through stereoscopic effect setting of the stimulus and shape setting of the stimulus, and may finely adjust the difficulty level through interval setting of the stimulus, background color setting of the stimulus, and border setting of the stimulus.

In this case, priorities of the interval setting of the stimulus, the background color setting of the stimulus, and the border setting method of the stimulus may be predicted through an artificial intelligence model based on previous training performance results of a plurality of trainees. That is, a priority to be applied to a trainee is determined through an artificial intelligence model trained to learn which difficulty adjustment method resulted in a good training effect according to previous training performance results of a plurality of trainees. Specifically, the artificial intelligence model may predict a priority suitable for a current state of the trainee in consideration of previous training performance results of other trainees and characteristics of the current trainee (age, degree of strabismus, training period, etc.).

As described above, after setting a basic difficulty level of training by setting a stereoscopic effect and a shape of a stimulus, the processor 15 may finely adjust a difficulty level of training in a manner suitable for a trainee by setting an interval, a background color, and a border of the stimulus according to the priority while the stereoscopic effect and the shape of the stimulus are fixed.

Meanwhile, the processor 15 may provide stereoscopic vision training by providing a third stimulus based on auditory sensation or tactile sensation in a form associated with the first stimulus (stereoscopic stimulus).

Specifically, the processor 15 may additionally provide a third stimulus below a threshold value (e.g., an auditory stimulus or a tactile stimulus below a level perceivable by a patient). By providing not only a visual stimulus but also stimuli of other senses together in this way, it is possible to increase accuracy of training while performing training having a higher difficulty level than when stereoscopic vision training is performed only through a visual stimulus.

Although FIG. 2 describes sequential execution of steps, this merely illustratively describes the technical spirit of the present embodiment, and thus a person having ordinary skill in the art to which the present embodiment pertains may variously modify and apply the present embodiment by changing an order described in FIG. 2 or executing the order in parallel within a range not departing from essential characteristics of the present embodiment, and thus FIG. 2 is not limited to a time-series order.

Meanwhile, in the above-described description, according to an implementation example of the present disclosure, steps described in FIG. 2 may be further divided into additional steps or may be combined into fewer steps. Also, some steps may be omitted as necessary, and an order between the steps may be changed.

Meanwhile, the disclosed embodiments may be implemented in a form of a recording medium storing instructions executable by a computer. The instructions may be stored in a form of program code, and when executed by a processor, may generate a program module to perform operations of the disclosed embodiments. The recording medium may be implemented as a computer-readable recording medium.

The computer-readable recording medium includes all types of recording media storing instructions readable by a computer. For example, the recording medium may include ROM (Read Only Memory), RAM (Random Access Memory), a magnetic tape, a magnetic disk, a flash memory, an optical data storage apparatus, and the like.

As described above, the disclosed embodiments have been described with reference to the accompanying drawings. A person having ordinary skill in the art to which the present disclosure pertains will understand that the present disclosure may be implemented in forms different from the disclosed embodiments without changing the technical spirit or essential characteristics of the present disclosure. The disclosed embodiments are illustrative and should not be interpreted as being restrictive.

## Claims

1. An apparatus for providing visual perception learning-based stereoscopic vision training, comprising:
a display module;
a memory configured to store at least one process for strabismus improvement based on stereoscopic vision training; and
a processor configured to generate a first stimulus plate for a normal eye among both eyes of a trainee and a second stimulus plate for a strabismic eye among the both eyes, and configured to provide the stereoscopic vision training through difficulty adjustment of a first stimulus among a plurality of stimuli included in each of the first stimulus plate and the second stimulus plate,
wherein the difficulty adjustment is performed through at least one of stereoscopic effect setting of a stimulus, shape setting of a stimulus, interval setting of a stimulus, border setting of a stimulus, and background color setting of a stimulus.

2. The apparatus of claim 1,
wherein the processor is configured to identify a strabismus angle of the strabismic eye through evaluation of a strabismus angle of the trainee, and configured to set positions of the first stimulus plate and the second stimulus plate within an image for the stereoscopic vision training based on the strabismus angle.

3. The apparatus of claim 2,
wherein the positions of the first stimulus plate and the second stimulus plate within the image are set such that a center of the first stimulus plate and a center of the second stimulus plate differ from each other by the strabismus angle on a horizontal line.

4. The apparatus of claim 1,
wherein the stereoscopic effect setting of the stimulus is a method in which a difficulty level is higher as a distance by which the first stimulus included in the second stimulus plate moves with respect to a center of the second stimulus plate is smaller, and
wherein the shape setting of the stimulus is a method in which a difficulty level is higher as a shape has less symmetry.

5. The apparatus of claim 4,
wherein the interval setting of the stimulus is a method in which a difficulty level is higher as a distance between the first stimulus and a second stimulus among the plurality of stimuli is greater, and
wherein the background color setting of the stimulus is a method in which a difficulty level is higher as a difference between the background color and colors of the first stimulus and the second stimulus is smaller.

6. The apparatus of claim 5,
wherein the border setting of the stimulus is a method in which a difficulty level is higher as a distance between a border set by a first method or a second method and at least one of the first stimulus and the second stimulus is greater,
wherein the first method comprises setting borders for the first stimulus and the second stimulus, respectively, and
wherein the second method comprises setting a border including the first stimulus and the second stimulus.

7. The apparatus of claim 6,
wherein the processor is configured to set a minimum border formation condition based on an angle for the stereoscopic effect setting, and configured to apply the minimum border formation condition when performing the border setting of the stimulus.

8. The apparatus of claim 1,
wherein the processor is configured to set priorities of the interval setting of the stimulus, the border setting of the stimulus, and the background color setting of the stimulus by using an artificial intelligence model trained with training results of other trainees.

9. The apparatus of claim 1,
wherein the processor is configured to provide the stereoscopic vision training by providing a third stimulus based on auditory sensation or tactile sensation in a form associated with the first stimulus.

10. A method for providing visual perception learning-based stereoscopic vision training performed by a processor of an apparatus, comprising:
generating, by the processor, a first stimulus plate for a normal eye among both eyes of a trainee and a second stimulus plate for a strabismic eye among the both eyes; and
providing, by the processor, stereoscopic vision training through difficulty adjustment of a first stimulus among a plurality of stimuli included in each of the first stimulus plate and the second stimulus plate,
wherein the difficulty adjustment is performed through at least one method of stereoscopic effect setting of a stimulus, shape setting of a stimulus, interval setting of a stimulus, border setting of a stimulus, and background color setting of a stimulus.
